# Europäisches Patentamt

# European Patent Office

# Office européen des brevets

(11) Publication number: **0 095 726**
**B1**

## EUROPEAN PATENT SPECIFICATION

(45) Date of publication of patent specification: **02.11.88**

(51) Int. Cl.⁴: $A\ 61\ N\ 1/38$, $A\ 61\ N\ 1/04$

(21) Application number: **83105192.5**

(22) Date of filing: **26.05.83**

(54) Apparatus for controlling cardiac ventricular tachyarrhythmias.

(30) Priority: **01.06.82 US 383781**

(43) Date of publication of application:
**07.12.83 Bulletin 83/49**

(45) Publication of the grant of the patent:
**02.11.88 Bulletin 88/44**

(84) Designated Contracting States:
**CH DE FR GB IT LI NL SE**

(56) References cited:
**DE-A-2 643 956**
**FR-A-2 257 312**
**US-A-3 359 984**
**US-A-3 605 754**
**US-A-4 088 140**

**Jan Kugelberg "Ventricular Defibrillation, New Aspect", Acta Chirurgica Scandinavica, Stockholm 1967**

**"The effect of shock separation time on multiple-shock defibrillation" by Moore et al (Medical Instrumentation, Vol. 12, No. 1, 1-2/1978)**

(73) Proprietor: **MEDTRONIC, INC.**
**7000 Central Avenue N.E.**
**Minneapolis Minnesota 55432 (US)**

(72) Inventor: **Tacker, Willis A., Jr.**
**2901 Wilshire Avenue**
**West Lafayette Indiana 47906 (US)**
Inventor: **Babbs, Charles Fredrick**
**804 E. Cumberland Avenue**
**West Lafayette Indiana 47906 (US)**
Inventor: **Bourland, Joe Dan**
**606 Wilshire Avenue**
**West Lafayette Indiana 47906 (US)**
Inventor: **Geddes, Leslie Alexander**
**400 North River Road, Apt. 1724**
**West Lafayette Indiana 47906 (US)**

(74) Representative: **Tomlinson, Kerry John et al**
**Frank B. Dehn & Co. European Patent Attorneys**
**Imperial House 15-19 Kingsway**
**London WC2B 6UZ (GB)**

(56) References cited:

**Optimization of Epicardial Electrode Size...,
Medical Instrumentation, Vol. 20, No. 1, Jan-
Feb 1986**

**Sequential Pulse Defibrillation..., Medical
Instrumentation, Vol. 20, No. 3, May-Jun 1986
Internal cardiac defibrillation in man..., Jones
et al, Circulation, Vol. 73, No. 3, March 1986
Influence of Cardiopulmonary Bypass...,
George Klein et al, The American Journal of
Cardiology, Vol. 57, May 1986**

## Description

This invention relates to the control of cardiac ventricular tachyarrhythmias, including fibrillation, and more particularly to an improved means which functions more effectively and at lower shock strength (voltage and current) than heretofore employed.

Ventricular fibrillation is an uncoordinated contraction and relaxation of the individual fibers of the heart which produces no blood flow and results in death unless corrective measures are applied within minutes of onset. Conventional treatment for the hospitalized patient calls for the application of an electric shock of sufficient strength voltage to depolarize most of the ventricular cells, e.g., by way of a pair of electrodes ("paddles") across the chest of the patient. For the patient outside the hospital who has been identified as a fibrillation risk, the shock can be applied to a pair of electrodes implanted in various ways around and within the heart, the shock being supplied by an implanted pulse generator in response to a command from a fibrillation or tachyarrhythmia detectors.

Of the various implanted electrode systems heretofore employed, the most efficient comprises a pair of electrodes placed on opposite sides of the heart in the epicardial-pericardial space. A system such as that disclosed in out contemporaneously filed application entitled Electrode System and Method for Implantable Defibrillators has numerous advantages over the prior-art systems, especially in avoiding toracotomy or other major surgery for implantation, and also in avoiding placement of the electrodes in the blood stream or in the heart thereby minimizing the problems of fibrosis, blood clot and embolus formation, and associated risk of stroke and infarction.

A fundamental limitation of all such electrode systems, however, is the current density not being uniform throughout the ventricles with the result that some regions of the venticles receive more current than necessary while other regions receive less. A larger total current is therefore required to achieve the "defibrillation threshold", and tissues in regions of high currents (usually those adjacent to the electrodes) are at a higher risk of sustaining damage.

In order to reduce the current sensity under the electrodes and at the same time to increase the volume of venticular tissue exposed to the current, we explored the possibility of increasing the electrode size, either by using wider electrodes or by extending them a greater distance along the epicardial wall from base to apex. In each case, however, before the desired result could be attained, the edges or ends of the electrodes approached each other closely enough to provide a shorter alternate path for the current through the epicardial tissue, thereby reducing rather than increasing the current through the ventricles, and increasing the risk of myocardial damage.

We have succeeded in avoiding the problems related to current density in a relatively simple manner by sequentially delivering two current pulses to separate pairs of electrodes implanted orthogonally to each other in the epicardial-pericardial space. Thus, we deliver two pulses to the ventricles, one in a lateral direction, and immediately before or afterward, another pulse in a ventral-dorsal direction. In this manner, we have achieved a temporal and spatial summation effect for the defibrillating current, resulting in a dramatic reduction in the voltage, current, and energy required to defribrillate when compared to all presently known electrode systems.

FR—A—2 257 312 discloses a defibrillator having a plurality of electrode pairs disposable around the heart, to which sequential pulses are supplied.

It is an object of the present invention to control cardia ventricular fibrillation and other tachyarrhythmias in an improved and more effective manner.

Another object is to maximize ventricular exposure in the control of fibrillation while minimizing current density.

Another object is to achieve cardiac ventricular defibrillation or cardioversion with less energy, lower voltage, and lower current.

A further object is to minimize the risk of myocardial damage in the control of ventricular fibrillation and other tachyarrhythmias.

A still further object is to minimize the size of implanted electrodes and implanted pulse generators used in the control of the cardiac ventricular fibrillation and other tachyarrhythmias.

The objects of the present invention are achieved by an implantable defibrillator as set out in claim 1. The technique produces a temporal and spatial summation of the delivered current, which results in a dramatic reduction in the voltage, current, and energy required for defibrillation or cardioversion, and allows the use of a smaller implantable pulse generator. The new system achieves a more uniform distribution of current within the ventricular myocardium, thereby reducing the risk of tissue damage and increasing the margin of safety between effective shocks and myocardial depressing or damaging shocks.

Other objects of the invention and its advantages over the prior art will be apparent from the following description of the accompanying drawings which illustrate and compare the invention with the prior art, and also show preferred embodiments exemplifying the best mode of carrying out the invention as presently perceived. In such drawings:

Fig. 1 is a simplified cross-section of the heart through the ventricles, looking upward, showing two laterally opposed electrodes in place on the epicardium;

Fig. 2 is similar to Fig. 1, but with addition of two ventrally-dorsally opposed electrodes, the adjacent electrodes being placed essentially orthogonal to each other;

Fig. 3 is similar to Fig. 1, but with multiple left-ventricle electrodes implanted;

Fig. 4 is a plan view of an electrode useful in the present invention;

Fig. 5 is a view in cross-section of the electrode of Fig. 4 at line 5—5;

Fig. 6 is a plan view of a cannula useful in the present invention;

Figs. 7 and 8 are cross-sectional views of the cannula of Fig. 6 at lines 7—7 and 8—8, respectively;

Fig. 9 is a cross-sectional view of the cannula of Fig. 6 showing the electrodes of Fig. 4 in place therein;

Fig. 10 is a frontal view of the human heart, partly in lateral cross-section, showing an endoscope inserted to the juncture of the pericardium and the diaphragm beneath the heart;

Fig. 11 is the same view as Fig. 10, with the cannula-electrode assembly of Fig. 9 in place in the pericardial space on the right side of the heart; and

Fig. 12 is the same view as Fig. 11 with the cannula and the endoscope removed, leaving the electrode in place in the right pericardial space.

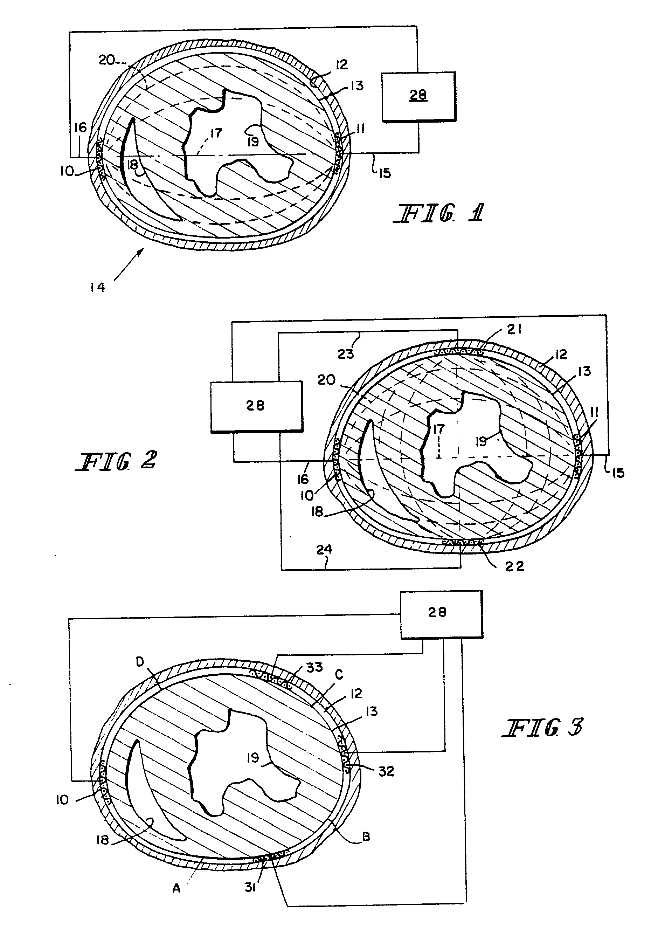

Fig. 1 illustrates an important limitation of conventional electrode systems using a single pair of electrodes, even when directly opposed, in that they do not provide a uniform current density throughout the ventricles. Two laterally opposed electrodes 10 and 11 implanted between the pericardial sac 12 and the epicardium 13 of the heart 14 are attached by way of leads 15 and 16 to a control device and pulse generator 28 of known design, such as, for example, any one disclosed in U.S. Patents 4,291,699; 4,184,493; or 4,202,340. When a pulse is applied to leads 15 and 16, the maximum current density lies mainly in the line 17 between the electrodes, passing through the tissues of the right ventricle 18 and the left ventricle 19, decreasing to a minimum near the heart wall 13 at line 20. Thus, relatively little current flows through the dorsal and ventral portions of the left ventricular tissue, and as a result a higher voltage and higher overall current density are required to achieve defibrillation or cardioversion.

In Fig. 2, a second pair of electrodes 21 and 22, placed dorsally and ventrally, are added to the arrangement of Fig. 1, with leads 23 and 24 connected to a pulse generator and controller (not shown) arranged to supply pulses in sequence to the lateral pair of electrodes and to the dorsal-ventral pair. The presence of the second pair of electrodes does not significantly alter the current distribution from the first pair, so long as the electrodes are relatively small with respect to the epicardial circumference, and so long as the two pairs of electrodes are isolated from each other during current flow. For this purpose, the distance between the adjacent edges of adjacent electrodes should not be smaller than the shorter of the distances between electrode pairs. As shown in Fig. 2, the current in the second pulse concentrates in the areas of low current density during the first pulse, with the result that the ventricular tissues are much more effectively treated, in particular the critically important left ventricle. Thus, the use of two pairs of electrodes implanted orthogonally permits the use of smaller electrodes, lower voltage and current, and lower total energy, which can be delivered by a smaller pulse generator, with less hazard of damage to the tissues adjacent the electrodes. For these reasons, the invention is safer and more effective than the prior art.

As a practical matter, our invention will normally employ two sets of opposed electrodes, one pair disposed laterally upon the epicardium and the other pair disposed ventrally-dorsally, each electrode being orthoganal to the adjacent electrodes. In principle, three or more electrodes can be used, if desired, spaced more or less evenly around the epicardium and pulsed sequentially, subject only to the limitation that they must not be so close together as to divert the current flow away from the ventricular tissues. For the typical fibrillation or tachycardia patient, however, we do not look upon such additional electrodes as either necessary or desirable, considering the additional surgery involved in installing them.

The location of the electrodes need not be orthogonal, but may vary somewhat, so long as the desired current flow through the left ventricular tissue is achieved. Indeed, because of the disparity in size between the ventricles (the left ventricle being much larger), an advantageous alternative arrangement is to install multiple left ventricular electrodes as shown in Fig. 3 with a single right ventricular electrode and to pulse each of the left ventricular electrodes in sequence, paired with the right ventricular electrode. Thus, in Fig. 3, the epicardium adjacent the left ventricle is divided into three equal segments represented by AB, BC, and CD, and an electrode 31, 32, 33 is implanted in the pericardial space at the middle of each segment. All of the electrodes are connected to a pulse generator and controller (not shown), which sequentially pairs electrodes 31, 32, and 33 with electrode 10 and delivers a pulse. In this way, maximum depolarization of the left ventricular tissues is achieved. With this arrangement, the only limit upon the number of left ventricular electrodes that may be used is the surgical trauma involved in installing them.

In carrying out the process of ventricular defibrillation or cardioversion according to the present invention, it is important to pulse the electrode pairs in sequence, with at least some time separation between the pulses. On the other hand, the time of separation should be limited, since we have observed that the threshold current for defibrillation increases with separation time, above about 2 milliseconds. As a general rule, we find that a separation period of about 0.1 millisecond to about 2 milliseconds is satisfactory, and we prefer about 0.5 millisecond.

For use in our invention, the truncated-exponential waveform suggested by Schuder et al,

*Trans. Am. Soc. Artif. Organs. 15*, 207—212, 1970, is the most practical type of pulse. In order to create the smallest pulse generator for implantation, it was necessary to determine the relationship between the capacitance of the energy-storage capaciator and the voltage to which it must be charged in order to deliver the minimum sufficient current to effect defibrillation. For this purpose, we conducted a study to determine the dependence of ventricular defibrillation threshold on the duration and tilt of the truncated-exponential waveform ("tilt" being defined as the percent decrease in current during the defibrillation pulse; e.g., if, at the end of the pulse, the defibrillating current has fallen to one-third its initial value, then the tilt is 67%). We found (1) that there exists a "strength-duration" curve for defibrillation, and (2) two waveforms of the same duration are equally effective if they have the same average current. The latter we have called the "Average Current Law" for defibrillation (Bourland et al, Medical Instrumentation 12: 42—45, 1978).

Generally, the relation between capacitance and voltage across the elements in a system in the absence of an external source of energy can be expressed by Kirchhoff's current law as:

$$C\frac{dV}{dt} + \frac{1}{R}V = 0$$

where
C is the capacitance of the system;
V is the voltage across the system;
R is the impedance of the system;

$\frac{1}{R}$ is the admittance of the system;

$\frac{dV}{dt}$ is the rate of change in the voltage V with respect to time.

It is also known that voltage V of the system at a particular time t can be expressed as follows:

$$V = V_o e^{-t/RC}$$

where
$V_o$ is the initial voltage of the system;
e is a constant approximately equal to 2.71828;
t is the displacement in time from the initial voltage.

The current, I, that flows in the defibrillating circuit also decreases exponentially with time (t) and is given by:

$$I = \frac{V_o}{R} e^{-t/RC}$$

Animal studies have shown (Bourland et al, Medical Instrumentation 12:42—45, 1978) that the current required to defibrillate is given by:

$$I = K_1 + K_2/d$$

where
d is the duration of a rectangular pulse used for defibrillation
$K_1$ and $K_2$ are constants which depend on the electrode system, patient species, and heart size.

The average current for a truncated-exponential waveform witht duration, d, is given by:

$$I = \frac{V_o}{Rd} \int_0^d e^{-t/RC} dt$$

$$I = \left[ -\frac{RC\, V_o}{Rd} e^{-t/RC} \right]_0^d$$

$$I = -\frac{CV_o}{d}(e^{-d/RC} - 1)$$

$$I = \frac{CV_o}{d}(1 - e^{-d/RC})$$

Combining these relationships and invoking the average current law for defibrillation, the voltage, $V_o$, to which the defibrillator must be charged, is given by:

$$K_1 + K_2/d = \frac{CV_o}{d}(1 - e^{-d/RC})$$

$$V_o = \frac{K_1 d + k_2}{C(1 - e^{-d/RC})}$$

Thus, the voltage $V_o$ to which the energy storage capacitor must be charged, if the generator losses are negligible, can be expressed as the following equation:

$$V_o = \frac{K_1 d + K_2}{C(1 - e^{-d/RC})}$$

The above equation permits selecting the smallest possible capacitor for use in an implantable defibrillator.

For use in our invention, pulses of truncated-exponential waveform of about 1 millisecond to about 5 milliseconds, applied to each electrode pair, are suitable preferably around 2.5 milliseconds, with appropriate separation between pulses as described above. Depending upon the electrode system employed, the delivered voltage will range between about 100 volts and about 1,000 volts, being around 200 and 400 volts in the preferred forms of our invention. The threshold defibrillation current ranges from about 5 amperes to about 10 amperes. The delivered energy for the preferred form of our invention ranges from one-third to two-thirds less than the threshold energy required for defibrillation in other systems.

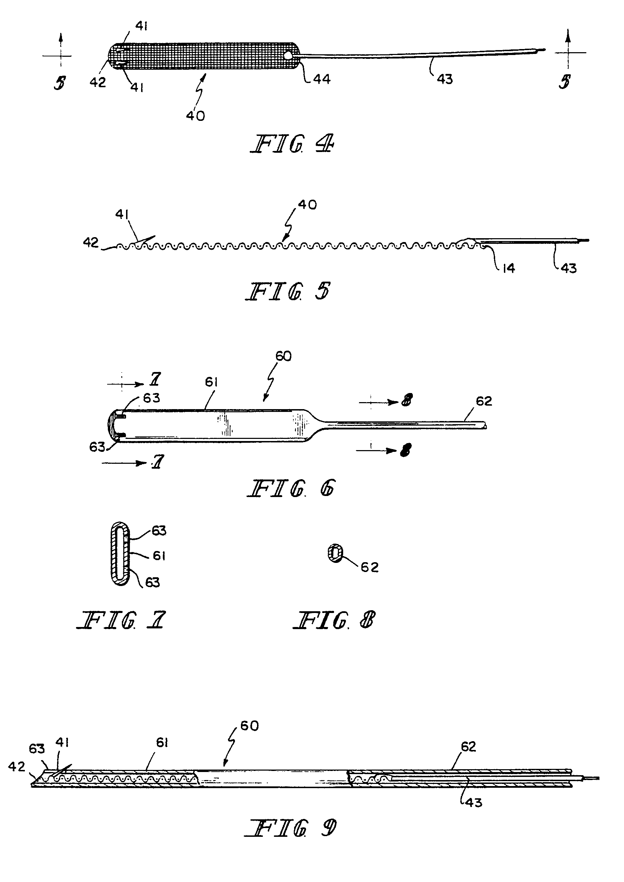

A preferred form of our invention uses two pairs of electrodes, implanted subpericardially by way of an endoscope, as illustrated in Figs. 4—12.

In Fig. 4, electrode 40 is a rectangular screen of titanium having one or more barbs 41 near one end 42 (the distal end when implanted) extending proximally, and an insulated lead wire 43 attached to the other end 44. The electrode 40 is inserted into the pericardial space with the barbs toward the pericardium, so that it becomes affixed to the pericardium by any reverse motion, such as the withdrawal of the cannula as described below.

Fig. 5 illustrates the electrodes 40 of Fig. 4 in cross-section at line 5—5, showing barb 41 near distal end 42 pointing proximally.

Fig. 6 illustrates a cannula 60 suitable for inserting the electrodes used in the present invention. The device is a tube, preferably made of a somewhat flexible substance such as polyethylene, flattened at its distal end 61 and optionally flattened to some extent at its proximal end 62 (See cross-sectional views at lines 7—7, Fig. 7, and 8—8, Fig. 8, respectively.) Slots 63 are provided in distal end 61 to accommodate and at least partially cover barbs 41 during insertion and to allow subsequent withdrawal of the cannula.

Fig. 9 shows cannula 60 in cross-section with electrode 40 lying in position within it, the barbs 41 extending into slots 63 and lead wire 43 protruding from proximal end 62.

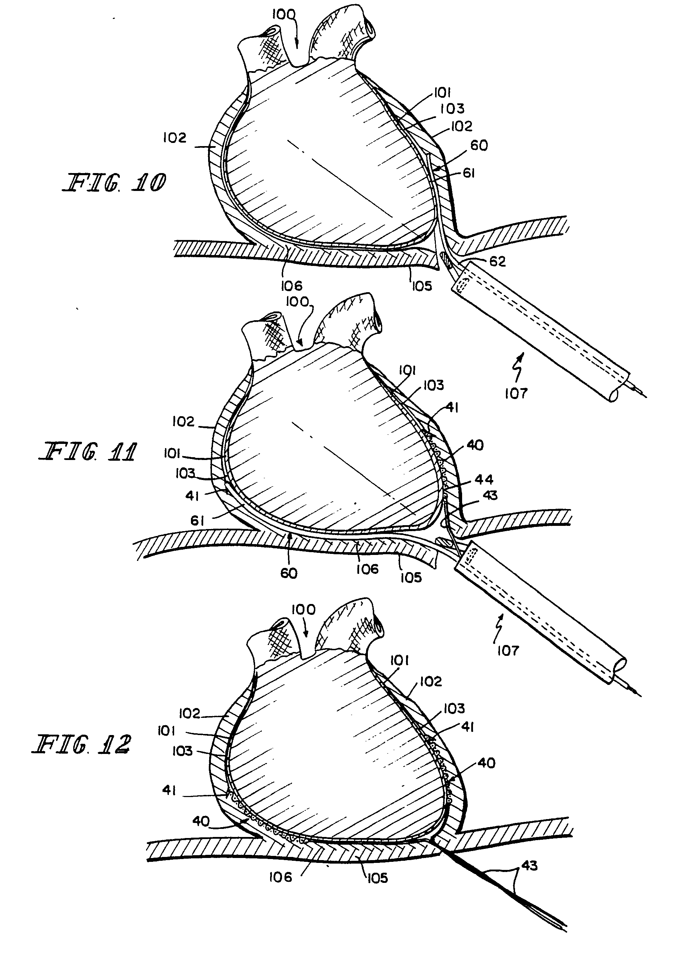

Figs. 10, 11, and 12 illustrate the preferred method for inserting electrodes for use in our invention.

Fig. 10 is a view of the heart 100 showing the outer surface 101 of the heart (the epicardium), the outer sac 102 enclosing the heart (the pericardium), the pericardial space 103 lying between them, the diaphragm 105 separating the thorax from the abdomen, the pericardium-diaphragm juncture 106 at which the two fuse into a single membrane, and an endoscope 107 having its distal end resting against membrane 106. The endoscope is of conventional design having two barrels, one fitted with a light and ptical means for observation, and the other open for insertion of operating devices. The endoscope is inserted into the abdominal cavity through an incision in the abdominal wall and is moved upward and rested against membrane 106 at a point beneath the left central portion of the heart. A small knife is inserted through the operating barrel and an incision is made in membrane 106 of a size to admit the distal end 61 of cannula 60. The knife is withdrawn and cannula 60, with electrode 40 and lead wire 43 in place, is inserted through the operating barrel and pushed forward, curving to the right and upward into the pericardial space on the right side of the heart as shown in Fig. 11. In this position, barbs 41 hook into the wall of the pericardium and retain electrode 40 in place when cannula 60 is withdrawn, as shown in Fig. 12.

A second electrode is implanted on the left side of the heart according to the same procedure, with appropriate manipulation of cannula 60 to start the motion of the cannula and electrode 40 toward the left side of the heart. In the same way, a third electrode is implanted ventrally and a fourth electrode dorsally.

The pulse generator and control unit 28 are implanted in a known way under the skin, usually of the abdomen or chest, and are connected internally to the lead wires. The device is then ready to function. Sensing devices in the control unit detect the onset of fibrillation or tachycardia and apply the necessary control pulses to the implanted electrodes, thereby restoring the normal heart beat.

The sequential pulsing used in our invention reduces the current required for defibrillation threshold and therefore allows electrodes of smaller size to be used safely. This is an advantage in the endoscopic method for implantation, which tends to limit the electrode width, more particularly because a smaller endoscope is preferred in order to minimize surgical trauma. Thus, the electrode width is usually around 5 to 10 mm. The electrode length is limited by the need to avoid having the electrodes too close together at the cardiac base and apex, which tend to short-circuit the electrical path. As noted above, the electrodes should preferably be no closer to each other at any point than the straight line through the heart between the pairs of electrodes. A suitable length is from about 10% to about 25% of the circumference of the heart. Depending upon the size of the heart, this will range from about 3 cm to about 9 cm.

**Claims**

1. An implantable defibrillator for controlling cardiac ventricular fibrillation or other tachyarrhythmias by passing an electric current through the heart, comprising a plurality of electrode pairs (10, 11, 21, 22) disposable around the heart and means (28) for sequentially pulsing the pairs, characterized in that each of the pairs of electrodes 10, 11, 21, 22) is subjected to pulses at a voltage between about 100 and about 400 volts, providing a current above the defibrillation threshold level, the pulses being separated by an interval of about 0.1 millisecond to about 2 milliseconds and having a duration of about 1 millisecond to about 5 milliseconds.

2. An apparatus according to claim 1 which comprises one pair (10, 11) of electrodes disposed laterally, another pair (21, 22) of electrodes disposed ventrally-dorsally and means (28) for sequentially pulsing each of said pairs of electrodes.

3. An apparatus according to claim 1 which comprises one electrode (10) disposed adjacent to the right ventricle, a plurality of spaced-apart electrodes (41, 42, 43) disposed adjacent to the left ventricle, and means (28) for sequentially pairing and pulsing the right ventricular electrode (10) with the left ventricular electrodes (41, 42, 43).

4. An apparatus according to claim 1 comprising three spaced-apart electrodes disposed adjacent the heart which are sequentially paired to provide two current pathways through the heart and means for sequentially pulsing each pair of electrodes by applying time-separated truncated-exponential pulses to each electrode pair.

## Patentansprüche

1. Implantierbarer Defibrillator zum Beherrschen von Herzkammerfilmmern oder anderen Tachyarrhythmien, indem ein elektrischer Strom über das Herz geleitet wird, mit einer Mehrzahl von um das Herz herum anordenbaren Elektrodenpaaren (10, 11, 21, 22) und Mitteln (28) zum sequentiellen Beaufschlagen der Paare mit Impulsen, dadurch gekennzeichnet, daß jedes der Elektrodenpaare (10, 11, 21, 22) Impulsen mit einer Spannung zwischen etwa 100 und etwa 400 Volt ausgesetzt ist, wobei für einen Strom oberhalb des Defibrillationsschwellwertes gesorgt wird, sowie die Impulse durch ein Interval von etwa 0,1 Millisekunden bis etwa 2 Millisekunden voneinander getrennt sind und eine Dauer von etwa 1 Millisekunde bis etwa 5 Millisekunden haben.

2. Vorrichtung nach Anspruch 1 mit einem lateral angeordneten Elektrodenpaar (10, 11) und einem weiteren Elektrodenpaar (21, 22), das ventral-dorsal angeordnet ist, sowie wie Mitteln (28) zum sequentiellen Beaufschlagen jedes der Elektrodenpaare mit Impulsen.

3. Vorrichtung nach Anspruch 1 mit einer Elektrode (10), die benachbart dem rechten Ventrikel angeordnet ist, einer Mehrzahl von in Abstand voneinander liegenden Elektroden (41, 42, 43), die benachbart dem linken Ventrikel angeordnet sind, und mit Mittel (28) zum sequentiellen Impulsbeaufschlagen und Paaren der dem rechten Ventrikel zugeordneten Elektrode (10) mit den dem linken Ventrikel zugeordneten Elektroden (41, 42, 43).

4. Vorrichtung nach Anspruch 1 mit drei in Abstand voneinander liegenden Elektroden, die benachbart dem Herz angeordnet sind und die sequentiell gepaart werden, um zwei Stromwege durch das Herz hindurch auszubilden, sowie mit Mitteln zum sequentiellen Impulsbeaufschlagen jedes Elektrodenpaares durch Anlegen von zeitlich getrennten stumpfexponentiellen Impulsen an jedes Elektrodenpaar.

## Revendications

1. Défibrillateur implantable pour combattre la fibrillation ventriculaire cardiaque ou autres tachyarthmies en faisant passer un courant électrique à travers le coeur, comprenant une multiplicité de paires d'électrodes (10, 11, 21, 22) disposables autour du coeur et des moyens (28) propres à pulser consécutivement les paires, caractérisé en ce que chacune des paires d'électrodes (10, 11, 21, 22) est soumise à des impulsions à une tension électrique comprise entre environ 100 et environ 400 volts, en fournissant un courant supérieur au niveau de seuil de défibrillation, les impulsions étant séparées par un intervalle d'environ 0,1 milliseconde à environ 2 millisecondes et ayant une durée d'environ 1 milliseconde à environ 5 millisecondes.

2. Appareil selon la revendication 1 qui comprend une paire (10, 11) d'électrodes disposées latéralement, une autre paire (21, 22) d'électrodes disposées ventrodorsalement et des moyens (28) propres à pulser consécutivement chacune desdites paires d'électrodes.

3. Appareil selon la revendication 1 qui comprend une électrode (10) disposée en adjacence au ventricule droit, une multiplicité d'électrodes mutuellement spacées (41, 42, 43) disposées en adjacence au ventricule gauche et des moyens (28) propres à apparier et à pulser concécutivement l'électrode venticulaire droite (10) avec les électrodes ventriculaires gauches (41, 42, 43).

4. Appareil selon la revendication 1, comprenant trois électrodes mutuellement espacées disposées en adjacence au coeur qui sont successivement appariées de manière à établir deux trajets de circulation de courant à travers le coeur et des moyens propres à pulser successivement chaque paire d'électrodes par application d'impulsions en exponentielle tronquée séparées dans le temps à chaque paire d'électrodes.

FIG. 1

FIG. 2

FIG. 3

0 095 726

FIG. 4

FIG. 5

FIG. 6

FIG. 7

FIG. 8

FIG. 9

2

FIG. 10

FIG. 11

FIG. 12